(19) Europäisches Patentamt
European Patent Office
Office européen des brevets

(11) Publication number: 0 091 767
B1

(12) **EUROPEAN PATENT SPECIFICATION**

(45) Date of publication of patent specification: **28.02.90**

(51) Int. Cl.[5]: **A 61 K 9/16**, A 61 K 47/00, A 23 K 1/16, A 23 K 1/17, A 23 K 1/175

(21) Application number: **83301890.6**

(22) Date of filing: **05.04.83**

(54) **Stabilization of unstable drugs or food supplements.**

(30) Priority: **05.04.82 US 365418**

(43) Date of publication of application:
**19.10.83 Bulletin 83/42**

(45) Publication of the grant of the patent:
**28.02.90 Bulletin 90/09**

(84) Designated Contracting States:
**AT BE CH DE FR GB IT LI LU NL SE**

(56) References cited:
**US-A-3 150 042**
**US-A-3 627 885**
**US-A-4 042 685**
**US-A-4 048 268**

(73) Proprietor: **MERCK SHARP & DOHME LTD.**
**Hertford Road**
**Hoddesdon Hertfordshire EN11 9NU (GB)**

(72) Inventor: **Yarwood, Richard J.**
**23 Grove Road**
**Stevenage Old Town Hertfordshire SG1 3NT (GB)**
Inventor: **Maxfield, Howard C.**
**20 Lansdowne Court Churchfields**
**Broxbourne Hertfordshire EN10 7JE (GB)**
Inventor: **Phillips, Anthony J.**
**Moredown Standon Road**
**Little Hadham Hertfordshire SG11 2DD (GB)**

(74) Representative: **Crampton, Keith John Allen et al**
**D YOUNG & CO 10 Staple Inn**
**London WC1V 7RD (GB)**

**EP 0 091 767 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the stabilization of drugs including certain antibiotics. More particularly and indeed exclusively, it concerns the granulation of Efrotomycin, milbemycins, tylosin and its derivatives, e.g. A.I.V. (3-acetyl-4″-isovaleryl tylosin), antibiotics B—5050 and tetrahydro-B—5050, Ivermectin, mocimycin, dihydromocimycin and goldinomycin. The present invention is based on the discovery that the granules obtained by granulating such compounds in alginic acid and magnesium hydroxide exhibit unexpectedly enhanced stability and can be incorporated into various formulations without substantial decomposition. When the drugs or food supplements are administered to animals, the formulations include animal feeds, pellets and feed premixes.

Efrotomycin (FR—02A) is a new antibiotic which also exhibits growth-promoting activity. It is effective against both gram-positive and gram-negative bacteria and accordingly is useful in the treatment of a broad spectum of infections in animals. Efrotomycin is dislcosed in US Patent US—A—4,024,251. The antibiotic is isolated from the fermentation broth of *Streptomyces lactamfuran* by solvent extraction and is believed to have the following molecular structure:

Efrotomycin (FR—02A) is a new antibiotic which also exhibits growth-promoting activity. It is effective against both gram-positive and gram-negative bacteria and accordingly is useful in the treatment of a broad spectrum of infections in animals. Efrotymycin is disclosed in U.S. Patent 4,024,251 issued 17 May 1977 to Maiese and Wax. The antibiotic is isolated from the fermentation broth of *Streptomyces lactamfuran* by solvent extraction and is believed to have the molecular structure as follows:

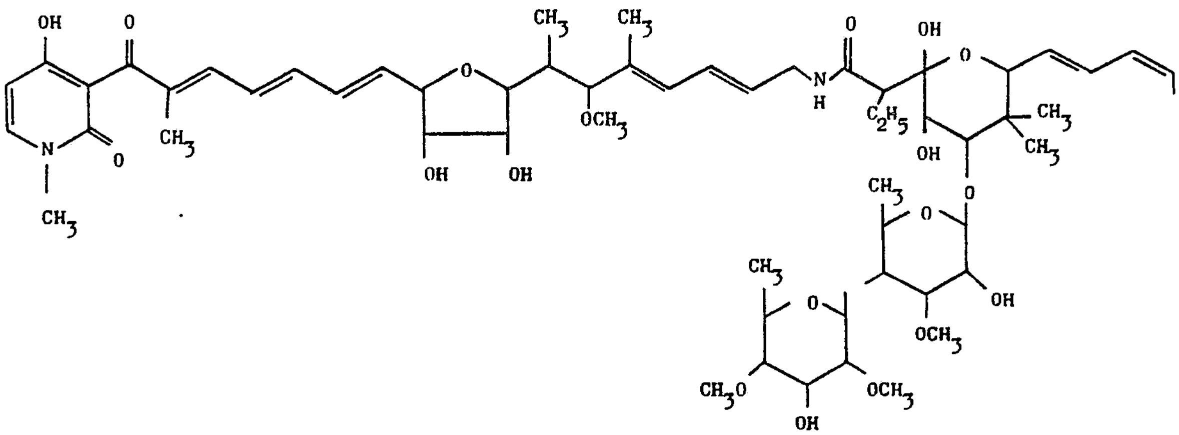

The physical properties of Efrotomycin (FR—02A) are summarized as follows:

Elemental analysis:

C 60.98%

H 7.60%

N 2.60%

The corresponding empirical formula $C_{59}H_{90}N_2O_{21}$ is consistent with monohydrated FR—02A. This is in agreement with a molecular weight of about 1168 of the sodium complex of FR—02A determined by field desorption mass spectrometry. Further mass spectroscopic study of FR—02A determined the molecular weight 1144 for the uncomplexed compound corresponding to the empirical formula $C_{59}H_{88}N_2O_{20}$.

FR—02A as the ammonium salt is soluble in alcohol and chloroform. It is moderately soluble in water at pH 7.0 or higher. A U.V. spectrum of the ammonium salt in water showed:

max. 233 nm: $E^{1\%}_{1cm} = 320$

max. 328 nm: $E^{1\%}_{1cm} = 180$

After further purification FR—02A in the free acid form has the following U.V. spectrum in methanol—0.05M phosphate buffer pH 6.85 (20:80):

max. 325 nm: $E^{1\%}_{1cm} = 317$

max. 230 nm: $E^{1\%}_{1cm} = 554$

max. 219 nm: $E^{1\%}_{1cm} = 556$

Specific optical rotation of FR—02A sodium salt is $[\alpha]^{20}_{D}$ −56.6 ± 0.5 (C=1, MeOH)

The nuclear magnetic resonance spectrum of antibiotic FR—02A was obtained at 100 MHz with $CDCl_3$ as the solvent and tetramethylsilane (TMS) as the internal standard. Representative features of the spectrum were Doublets at 1.21(3H), 1.31(3H), 1.74(3H), 4.63(1H), 4.87(1H), 5.94(1H) and 7.32(1H) ppm. Overlapping signals of 4 other C-methyl groups centered at about 0.94 ppm; Singlets at 1.65(3H), 2.02(3H), 3.15(3H), 3.42(3H), 3.45(3H), 3.54(3H), and 3.58(3H) ppm.

The infrared absorption spectrum of antibiotic FR—02A in a Nujol mull exhibits characteristic absorption at the following wave lengths expressed in reciprocal centimeters:

Broad Band at: 3400

Strong bands at: 1640, 1460, 1380, 1080, 1020

Prominent bands at: 1550, 1505, 1240, 1195, 940, 860, 720, 620.

Further characteristics of FR—02A as well as the process for isolating the antibiotic are described in U.S. Patent 4,024,251.

Efrotomycin is found to be unstable at elevated temperatures especially in the presence of moisture and feed components. However, in administering Efrotomycin to animals, it is most convenient and economic to include the antibiotic-growth promoter agent in premixes for animal feeds. Usually a premix is blended into animal feeds followed by injection of steam resulting in a final temperature of 85—100°C. The mixing process takes 2—15 minutes. The agglomerates may be either cooled and dried to produce a mash feed or extruded to give pelleted feed. In other words, Efrotomycin must be stabilized before it can be incorporated into animal feeds.

Accordingly, it is desirable to develop a method of formulation for the stabilization of Efrotomycin to enable its inclusion in animal feed.

One of the commonly used methods of formulation for stabilization is granulation because of ease, efficiency and consequently lower cost. Methods described in the literature include granulation with specific inorganic materials (United States Patent US—A—3,627,885) or with starch (US—A—4,048,268). Neither of these techniques is suitable for Efrotomycin.

Granulation with inorganic salts, particularly those of magnesium, did result in some stabilization but unexpected synergistic improvement occurred when polysaccharides were incorporated into the formulation as can be seen from Table 1.

Table 1. The effect of the addition of polysaccharide gelling agents to magnesium hydroxide on the stability of efrotomycin stored in animal feed at 50°C, (all contain 5% efrotomycin and magnesium hydroxide:gum in the weight ratio 1:1).

| Polysaccharides | Magnesium hydroxide | Storage time | % of initial remaining |
|---|---|---|---|
| — | — | 17 days | 12 |
| — | present | 25 days | 56 |
| Guar gum (anionic) | present | 28 days | 71 |
| Guar gum (nonionic) | present | 28 days | 79 |
| Guar gum (cationic) | present | 28 days | 55 |
| Tragacanth | present | 28 days | 68 |
| Acacia | present | 28 days | 81 |
| Alginic acid | present | 35 days | 100 |
| Calcium alginate | present | 56 days | 82 |
| Sodium alginate | present | 30 days | 69 |
| Maize starch | present | 14 days | 90 |
| Locust Bean gum | present | 14 days | 83 |
| Agar-agar | present | 14 days | 80 |

For efrotomycin, incorporation of alginic acid gives the best stabilization although all the polysaccharides listed in Table 1 offer significant protection.

In case of efrotomycin, the ratio of alginic acid to magnesium hydroxide is important as can be seen in Table 2.

Table 2. The effect of alginic acid — magnesium hydroxide ratio of the stability of efrotomycin stored in animal feed at 50°C (all contain 10% efrotomycin).

| % Magnesium hydroxide w/w | % Alginic acid w/w | % of initial remaining after 4 months storage |
|---|---|---|
| 90 | — | 26 |
| 75 | 15 | 73 |
| 60 | 30 | 91 |
| 45 | 45 | 93 |
| 30 | 60 | 100 |
| 15 | 75 | 75 |
| — | 90 | 37 |

It should be noted that the method of the present invention is not limited to Efrotomycin. Any other unstable animal drugs or food supplements may be incorporated into animal feeds or other formulations including human drug formulations according to the formula and process described herein. Particularly, for example, the following drugs:

(1) Ivermectin: a potent antiparasitic agent disclosed in U.S. Patent 4,199,569.

(2) Milbemycins (antibiotics B—41): antibiotics characterized in U.S. Patent 4,144,352; 3,950,360; and British Patent Specification No. 2,056,986.

(3) Tylosin and derivatives, e.g., A.I.V.: antibiotics disclosed in U.S. Patent 4,092,473. A.I.V. is the 3-acetyl-4''-isovaleryl derivative ($R_1$ is acetyl and R is isovaleryl in formula I) of tylosin.

(4) Antibiotics B—5050 and tetrahydro-B—5050: disclosed in U.S. Patent 3,853,842.

(5) Mocimycin and dihydromocimycin, antibacterial agents disclsoed in U.S. Patents 3,927,211 and 4,062,948.

(6) Goldinomycin disclosed in U.S. Patent 3,657,421.

The physical characterization, the biological activity and the isolation of the above-identified drugs are disclosed in the above documents.

It has been found that these drugs may also be stabilized by granulation with a polysaccharide gelling agent especially alginic acid blended with an inorganic salt, particularly metal oxides or hydroxides such as magnesium hydroxide. The granules may be incorporated into feed, tablets, capsules, or other formulations.

The present invention concerns a method of granulation for the stabilization of the unstable or heat-sensitive animal drugs Efrotomycin, tylosin and its derivatives (e.g. A.I.V.), milbemycins, antibiotics B—5050 and tetrahydro-B-5050, Ivermectin, mocimycin, dihydromocimycin, and goldinomycin. The granulation enables the incorporation of these drugs or food supplements into animal feeds or other formulations without substantial decomposition due to heat, humidity, and other adverse conditions. By proceeding in accordance with the invention, and in particular the preferred embodiments set out below, it is possible to produce sufficiently stable granules for inclusion of unstable drugs or food supplements in animal feeds, other human and animal formulations such as feed, tablet and capsules.

The present invention provides a stable granular formation for administering a compound in feed components, comprising:

(a) 0.1 to 70 parts by weight of Efrotomycin, Ivermectin, a milbemycin, tylosin or a derivative thereof, antibiotic B—5050, antibiotic tetrahydro-B—5050, mocimycin, dihydromocimycin or goldinomycin, or a combination of two or more of them;

(b) 10 to 80 parts by weight of alginic acid or a salt thereof; and

(c) 10 to 80 parts by weight of magnesium hydroxide.

In a preferred embodiment, the formula comprises:

(a) 2—40 parts by weight of the active compound;

(b) 20—50 parts by weight of alginic acid or its salt, preferably calcium alginate, or a combination thereof in the ratio 2—3 parts of alginic acid to 2—3 parts of calcium alginate; and

(c) 20—85 parts by weight of a metal oxide or hydroxide.

In the especially preferred embodiment of this invention the formula comprises:

(a) 5—35 parts by weight of an active compound;

(b) 15—50 parts by weight of alginic acid; and

(c) 20—80 parts by weight of magnesium hydroxide.

Efrotomycin, while it is unstable in the below-described feeds and feed additives, does not appear to be unstable to water alone. Thus, the process of the invention is not a strict protection method against

hydrolysis. The formulation of the invention protects antibiotics against deterioration in the presence of feeds. The Applicants do not wish to be bound by theory, but it may be that this protection results from isolation of the compound from the components of the feed that cause the deterioration. Thus any of the specified compounds intended for use in feed or feed-like components, which are unstable in such feeds or feed-like components but otherwise stable under neutral conditions, will benefit from the use of the process of this invention.

For preparing the above defined formulae, the active compound is mixed and agglomerated with other ingredients in the indicated amounts. A sufficient amount of a solvent, for example water, a $C_{1-6}$ alkanol such as ethanol or methanol, or a $C_{1-6}$alkanone such as acetone or diethylketone, or a mixture thereof, is added and thoroughly dispersed to obtain a wet mass of the desired consistency. Usually the amount of the solvent needed is 0.05—2 volumes per volume of the mixed ingredients. Subsequently, the wet blend is sieved, dried, and screened to yield granules of desired sizes. Alternatively, the mixing can be carried out in a high speed mixer-granulator followed by milling and drying in a fluidized bed.

Alternatively, the granulated product defined above may also be obtained by dry compression of the ingredients in the indicated amounts followed by subsequent grinding in order to get the granulated product. Alternatively, the mixed ingredients may be slurried with a suitable solvent and spray dried into granules.

The amount of biologically active compound in the granules may be adjusted up to the most convenient range — e.g., from 0.1 percent to 70 percent by weight — for facilitating the dispersion of the compounds in the feed, and the resulting composition (granules) is then dispersed in any suitable feed, premix substrate or simply used as premix by itself. When the granules are dispersed in animal feed, it is usually incorporated at the rate of about 0.1—10 kg and preferably 0.5—2 kg per metric ton to achieve the desired dose.

Usually the wet-granulation technique is used, the active compound, for example, Efrotomycin, is thoroughly mixed in the indicated amount with alginic acid and magnesium hydroxide. An adequate amount of water or other solvent is added to obtain a wet mass of required consistency. The resulting agglomerate is then granulated by passing through a 16 mesh (1000 μm) screen and dried at about 30°—60°C, preferably at about 45°C for about 5—48 hours, usually about 15—20 hours. Optionally, the granules may be rescreened through a 30 mesh (595 μm) or other suitable screen to obtain the required size.

Alternatively the mixing can be carried out in a high speed mixer granulator followed by milling and drying in a fluidized bed at about 30°C to 55°C for about 1—5 hours.

Although it is not required for performing the invention the formulation may be admixed with suitable inert diluents such as lactose, sucrose, calcium phosphate or micro-crystalline cellulose. Disintegrating agents (e.g. starch or its modifications) or lubricants such as magnesium stearate, stearic acid, polyethylene glycol or talc may be added. The blend may be filled into capsules or compressed into tablets to allow the administration of stabilized drugs, e.g., Ivermectin, as a convenient oral dose.

The following examples are intended to illustrate the preparation of compositions of the invention.

### Example 1

A wet blend was prepared from mixing the following components:

| | |
|---|---|
| Efrotomycin (60% pure) | 33.33 parts by weight |
| Alginic acid | 13.33 parts by weight |
| Magnesium hydroxide | 53.34 parts by weight |
| Water | sufficient to granulate |

The wet blend was sieved 16 mesh, dried at 45°C for 2 hours and then rescreened 30 mesh.

The dried granule was used in a "concentrate" which may then be blended with other inert ingredients, e.g., oiled rice hulls and then incorporated into animal feed at the rate of 0.5—2 kg per ton to achieve the appropriate dose. The stabilization of Efrotomycin was achieved in both the premix and feed as shown below in Table III.

TABLE III

Stability of unprotected and protected Efrotomycin (100 ppm) in feed and pelleted feed. (Concentrate contains 20% by weight Efrotomycin; means ± 1 std. deviation)

| Storage Conditions | | Stability in feed (w/w% initial) | | Stability in pelleted feed (w/w% initial) |
|---|---|---|---|---|
| | | Efrotomycin (60% pure) | Concentrate | Concentrate |
| 2 wks | 40°C | — | — | 90.4 ± 13.1 |
| | 50°C | — | 87.3 ± 13.3 | 80.7 ± 11.8 |
| 17 days | 40°C | 22.1 ± 4.5 | — | — |
| | 50°C | 11.9 ± 3.3 | — | — |
| 4 wks | 40°C | 16.5 ± 6.3 | 75.0 ± 8.1 | 88.5 ± 5.7 |
| | 50°C | Trace | 73.1 ± 13.3 | 66.5 ± 4.7 |
| 6 wks | 40°C | 10.5 ± 3.2 | 74.6 ± 4.2 | 98.2 ± 10.2 |
| | 50°C | Trace | 78.8 ± 8.1 | 64.3 ± 3.67 |
| 12 wks | 40°C | — | 106 ± 12.7 | 75.0 ± 8.1 |

Following substantially the same procedure as described above, but substituting for Efrotomycin used therein Ivermectin, there is prepared a stabilized concentrate of Ivermectin.

Example 2

A wet blend was prepared from mixing the following components.

| | |
|---|---|
| Efrotomycin (60% pure) | 8.35 parts by weight |
| Alginic acid | 18.33 parts by weight |
| Magnesium hydroxide | 73.32 parts by weight |
| Water | sufficient to granulate |

The wet blend was treated as described in Example 1 and the stabilization achieved in feed is shown below.

| Storage conditions | | Stability in feed (w/w% initial) |
|---|---|---|
| 4 wks | 40°C | 91.3 ± 7.9 |
| | 50°C | 81.9 ± 8.7 |
| 7 wks | 40°C | 89.8 ± 8.6 |
| | 50°C | 72.1 ± 0.5 |
| 12 wks | 40°C | 96.0 ± 9.6 |

Following substantially the same procedure as described above, but substituting for Efrotomycin used therein Ivermectin, there is prepared a stabilized concentrate of Ivermectin.

Example 3

A wet blend was prepared by mixing the following components.

| | |
|---|---|
| Efrotomycin | 8.4 parts by weight |
| Alginic acid | 45.8 parts by weight |
| Magnesium hydroxide | 45.8 parts by weight |
| Water | sufficient to granulate |

The wet blend was treated as described in Example 1 and the stabilization in feed is shown below.

| Storage Conditions | Stability in feed (% initial) |
|---|---|
| 9 weeks at 50°C | 99 ± 9 |

Example 4

A wet blend was prepared by mixing the following components.

| | |
|---|---|
| Efrotomycin | 8.4 parts by weight |
| Calcium alginate | 45.8 parts by weight |
| Magnesium hydroxide | 45.8 parts by weight |
| Water | sufficient to granulate |

The wet blend was treated as described in Example 1 and the stabilization in feed is shown below.

| Storage Conditions | Stability in feed (w/w% initial) |
|---|---|
| 4 weeks at 50°C | 83 ± 4 |
| 8 weeks at 50°C | 82 ± 5 |

Example 5

A wet blend is prepared by mixing the following components.

| | |
|---|---|
| Efrotomycin | 8.4 parts by weight |
| Calcium alginate | 22.9 parts by weight |
| Alginic acid | 22.9 parts by weight |
| Magnesium hydroxide | 45.8 parts by weight |
| Water | sufficient to granulate |

The wet blend is treated as described in Example 1.

Example 6

A wet blend was prepared by mixing the following components.

| | |
|---|---|
| Efrotomycin | 8.4 parts by weight |
| Maize starch | 45.8 parts by weight |
| Magnesium hydroxide | 45.8 parts by weight |
| Water | sufficient to granulate |

The wet blend was treated as described in Example 1 and the stabilization in feed is shown below.

| Storage Conditions | Stability in feed (w/w% initial) |
|---|---|
| 14 days at 50°C | 90 ± 8 |
| 28 days at 50°C | 83 ± 9 |
| 56 days at 50°C | 67 ± 8 |

Example 7

A wet blend was prepared by mixing the following components.

| | |
|---|---|
| Efrotomycin | 8.4 parts by weight |
| Alginic acid | 45.8 parts by weight |
| Magnesium oxide | 45.8 parts by weight |
| Water | sufficient to granulate |

The wet blend was treated as described in Example 1 and the stabilization in feed is shown below.

| Storage Conditions | Stability in feed (w/w% initial) |
|---|---|
| 18 days at 50°C | 94 ± 2 |
| 56 days at 50°C | 83 ± 2 |
| 5 months at 50°C | 82 ± 4 |

Example 8

A wet blend was prepared by mixing the following components.

| | |
|---|---|
| Efrotomycin (60% pure) | 33.33 parts by weight |
| Alginic acid | 33.33 parts by weight |
| Magnesium hydroxide | 33.33 parts by weight |
| Water | sufficient to granulate |

The wet blend was treated as described in Example 1 and the stabilization in feed is shown below.

| Storage Conditions | Stability (%) |
|---|---|
| *In Mash* | |
| 12 weeks at 37°C | 93 ± 6 |
| 12 weeks at 37°C (Sodium Salt) | 113 ± 7 |
| *Pellets* | |
| 12 weeks at 37°C | 84 ± 11 |
| 12 weeks at 37°C (Sodium Salt) | 93 ± 8 |

Example 9

A wet blend was prepared by mixing the following components.

| | |
|---|---|
| Ivermectin | 1 part by weight |
| Alginic acid | 49.5 parts by weight |
| Magnesium hydroxide | 49.5 parts by weight |
| Water | sufficient to granulate |

The wet blend was treated as described in Example 1 and the stabilization in feed is shown below.

| Storage Conditions | Stability in feed (w/w% initial) | |
|---|---|---|
| | Ivermectin | Protected Ivermectin |
| 7 days at 40°C | 90 | — |
| 14 days at 40°C | 82 | — |
| 4 weeks at 50°C | — | 85 |

Example 10

A blend is prepared by mixing the following components.

| | |
|---|---|
| Ivermectin | 2 parts by weight |
| Alginic acid | 32.5 parts by weight |
| Starch (Directly compressible) | 32.5 parts by weight |
| Magnesium hydroxide | 32.5 parts by weight |
| Magnesium stearate | 0.5 parts by weight |

The blend is then compressed on a suitable tablet machine to produce thin compacts which are then milled to produce granules of about 0.5 mm diameter. Alternatively the blend may be passed through a roller compacter followed by screening.

The granule is then incorporated into feed as described in Example 1.

Example 11

A wet blend is prepared by mixing the following components.

| | |
|---|---|
| A.I.V. | 20 parts by weight |
| Alginic acid | 40 parts by weight |
| Magnesium hydroxide | 40 parts by weight |
| Water | sufficient to granulate |

The wet blend is treated as described in Example 1.

Example 12

Preparation of Tablet Formulation

| Ingredient | Milligrams Per Tablet |
|---|---|
| Ivermectin granule | 1.5 |
| Bone meal flour | 300 |
| Microcrystalline cellulose | 500 |
| Flavor | 250 |
| Dibasic calcium phosphate | 739.5 |
| Magnesium stearate | 9 |

The active granule is blended with a portion of the dibasic calcium phosphate and then incorporated with the flavor, microcrystalline cellulose and bone meal flour. The mix is blended to ensure homogeneity of Ivermectin, the magnesium stearate added and mixing continued for 3 minutes before compression on a suitable machine. Each tablet contains 75 µg of Ivermectin.

Example 13

Preparation of Capsule Formulation

| Ingredient | Milligrams per Capsule |
|---|---|
| Ivermectin granule as prepared in Example 9 | 10 |
| Starch | 109 |
| Magnesium Stearate | 1.0 |

The active ingredient, starch and magnesium stearate are blended together. The mixture is used to fill hard shell gelatin capsules of a suitable size at a fill weight of 120 mg per capsule.

Example 14

Following the procedure of Example 1, a protected wet blend containing mocimycin was prepared. The protected wet blend was granulated and incorporated into mash or feed pellets containing 100 ppm of mocimycin. The stability was noted as follows (percentages of original after the indicated time period):

| | | | |
|---|---|---|---|
| In mash | 6 weeks | at 30°C | 100% |
| | 6 weeks | at 37°C | 99% |
| In pellets | 6 weeks | at 30°C | 96% |
| | 6 weeks | at 37°C | 83% |

The unprotected drug has a stability of less than 25% after 2 months at 37°C.

Example 15

Following the procedure of Example 1 a protected wet blend containing goldinomycin was prepared. The protected wet blend was granulated and incorporated into feed. The stability is rated as follows:

| Storage Conditions | Stability (%) |
|---|---|
| 6 weeks at 37°C | 97% |

**Claims**

1. A stable granular formation for administering a compound in feed components, comprising:
(a) 0.1 to 70 parts by weight of Efrotomycin, Ivermectin, a milbemycin, tylosin or a derivative thereof, antibiotic B—5050, antibiotic tetrahydro-B—5050, mocimycin, dihydromocimycin or goldinomycin, or a combination of two or more of them;
(b) 10 to 80 parts by weight of alginic acid or a salt thereof; and
(c) 10 to 80 parts by weight of magnesium hydroxide.

2. A formulation as claimed in Claim 1 in which the amount of ingredient (a) is from 0.1 to 70% by weight of the granular formulation.

3. A formulation as claimed in Claim 1 or 2 in which the ratio is
(a) 2—40 parts by weight of ingredient (a);
(b) 20—50 parts by weight of the alginic acid or its salt;
(c) 20—85 parts by weight of magnesium hydroxide.

4. A formulation as claimed in Claim 3 in which the ratio by weight is
(a) 5—35 parts of ingredient (a);
(b) 15—50 parts of the alginic acid;
(c) 20—80 parts of magnesium hydroxide.

5. A formulation as claimed in Claim 4 in which the ratio by weight is
(a) 5 parts of the ingredient (a);
(b) 47.5 parts of the alginic acid; and
(c) 47.5 parts of magnesium hydroxide.

6. A formulation as claimed in any preceding claim in which ingredient (a) is Efrotomycin.

7. A formulation as claimed in any preceding claim in which the polysaccharide gelling agent is alginic acid, calcium alginate, starch or a mixture thereof.

8. A process for preparing a stable granular formulation as claimed in Claim 1 that comprises
(1) preparing a wet blend of the components described in sections (a), (b) and (c) of Claim 1 by mixing with water, a lower alkanol, a lower alkanonone or a mixture of two or more of them;
(2) drying the blend; and
(3) preparing granules of desired sizes from the blend.

9. A process as claimed in Claim 8 in which the component (a) is Efrotomycin, Ivermectin, 3-acetyl-4″-isovaleryl tylosin, mocimycin, or goldinomycin.

10. A process as claimed in Claim 8 or 9 in which the ratio of the components is as defined in any one of Claims 3 to 5.

11. A pharmaceutical composition comprising a pharmaceutically acceptable carrier and an effective amount of a granular formation according to any one of Claims 1 to 7.

12. A composition as claimed in Claim 11 in which the carrier is an animal feed.

**Patentansprüche**

1. Eine stabile, körnige Formulierung zur Verabreichung einer Verbindung in Nahrungskomponenten, enthaltend:
(a) 0,1 bis 70 Gew.-Teile von Efrotomycin, von Ivermectin, einem Milbemycon, von Tylosin, oder von einem Derivat davon, von Antibiotikum B—5050, von Antibiotikum Tetrahydro-B—5050, von Mocimycin,

von Dihydromocimycin oder von Goldinomycin, oder einer Kombination von zwei oder mehreren davon;

(b) 10 bis 80 Gew.-Teile von Alginsäure oder einem Salz davon; und

(c) 10 bis 80 Gew.-Teile von Magnesiumhydroxid.

2. Eine Formulierung wie in Anspruch 1 beansprucht, wobei die Menge des Bestandteils (a) 0,1 bis 70%, bezogen auf das Gewicht der körnigen Formulierung, ausmacht.

3. Eine Formulierung wie in Anspruch 1 oder 2 beansprucht, wobei das Verhältnis wie folgt ist:

(a) 2—40 Gew.-Teile von Bestandteil (a);

(b) 20—50 Gew.-Teile von Alginsäure oder ihrem Salz;

(c) 20—85 Gew.-Teile von Magnesiumhydroxid.

4. Eine Formulierung wie in Anspruch 3 beansprucht, wobei das Gewichtsverhältnis wie folgt ist:

(a) 5—35 Teile von Bestandteil (a);

(b) 15—50 Teile der Alginsäure;

(c) 20—80 Teile von Magnesiumhydroxid.

5. Eine Formulierung wie in Anspruch 4 beansprucht, wobei das Gewichtsverhältnis wie folgt ist:

(a) 5 Teile des Bestandteils (a);

(b) 47,5 Teile der Alginsäure; und

(c) 47,5 Teile von Magnesiumhydroxid.

6. Eine Formulierung wie in einem der vorhergehenden Ansprüche beansprucht, wobei der Bestandteil (a) Efrotomycin ist.

7. Eine Formulierung wie in einem der vorhergehenden Ansprüche beansprucht, wobei das Polysaccharidgeliermittel Alginsäure, Calciumalginat, Stärke, oder ein Gemisch davon, ist.

8. Eine Verfahren zur Herstellung einer stabilen, körnigen Formulierung, wie in Anspruch 1 beansprucht, welches umfaßt.

(1) das Herstellen eines feuchten Gemisches der in den Abschnitten (a), (b) und (c) von Anspruch 1 beschriebenen Komponenten durch Vermischen mit Wasser, einem niedrigen Alkanol, einem niedrigen Alkanon, oder einem Gemisch von zweien oder mehreren davon;

(2) das Trocknen des Gemisches; und

(3) das Herstellen von Körnern der gewünschten Größen aus dem Gemisch.

9. Ein Verfahren wie in Anspruch 8 beansprucht, wobei die Komponente (a) Efrotomycin, Ivermectin, 3-Acetyl-4''-isovaleryltylosin, Mocimycin oder Goldinomycin ist.

10. Ein Verfahren wie in Anspruch 8 oder 9 beansprucht, wobei das Verhältnis der Komponenten wie in einem der Ansprüche 3 bis 5 definiert ist.

11. Eine pharmazeutische Zusammensetzung, enthaltend einen pharmazeutisch annehmbaren Träger und eine wirksame Menge einer körnigen Formulierung nach einem der Ansprüche 1 bis 7.

12. Eine Zusammensetzung wie in Anspruch 11 beansprucht, wobei der Träger ein Tierfutter ist.

**Revendications**

1. Formulation granulaire stable permettant d'administrer un composé dans des constituents alimentaires, comprenant:

(a) 0,1 à 70 parties en poids d'Efrotomycine, d'Ivermectine, d'une milbémycine, de tylosine ou d'un de ses dérivés, d'un antibiotique B—5050, d'un antibiotique tétrahydro-B—5050, de mocimycine, de dihydromocimycine ou de goldinomycine, ou d'une combinaison de deux ou plus de deux d'entre eux;

(b) 10 à 80 parties en poids d'acide alginique ou d'un de ses sels; et

(c) 10 à 80 parties en poids d'hydroxyde de magnésium.

2. Formulation selon la revendication 1, dans laquelle la quantité d'ingrédient (a) est de 0,1 à 70% du poids de la formulation granulaire.

3. Formulation selon la revendication 1 ou 2, dans laquelle les proportions sont de:

(a) 2—40 parties en poids d'ingrédient (a);

(b) 20—50 parties en poids d'acide alginique ou de son sel;

(c) 20—85 parties en poids d'hydroxyde de magnésium.

4. Formulation selon la revendication 3, dans laquelle les proportions pondérales sont de:

(a) 5—35 parties en poids d'ingrédient (a);

(b) 15—50 parteis en poids d'acide alginique;

(c) 20—80 parties en poids d'hydroxyde de mangésium.

5. Formulation selon la revendication 4, dans laquelle les proportions pondérales sont de:

(a) 5 parties en poids d'ingrédient (a);

(b) 47,5 parties en poids d'acide alginique; et

(c) 47,5 parties en poids d'hydroxyde de magnésium.

6. Formulation selon l'une quelconque des revendications précédentes, dans laquelle l'ingrédient (a) est l'Efroto-mycine.

7. Formulation selon l'une quelconque des revendications précédentes, dans laquelle l'agent gélifiant polysaccharide est l'acide alginique, l'alginate de calcium, l'amidon ou un mélange de ceux-ci.

8. Procédé de préparation d'une formulation granulaire stable selon la revendication 1, qui comprend:

(1) la préparation d'un mélange humide des constituants décrits dans les sections (a), (b) et (c) de la

revendication 1, par mélange avec de l'eau, un alcool inférieur, une alcanone inférieure ou un mélange de deux ou plus de deux d'entre eux.

(2) la séchage du mélange; et

(3) la préparation de granules de dimensions souhaitées à partir du mélange.

9. Procédé selon la revendication 8, dans laquel le constituant (a) est l'Efrotomycine, l'Ivermectine, la 3-acétyl-4''-isovaléryltylosine, la mocimycine ou la goldinomycine.

10. Procédé selon la revendication 8 ou 9, dans lequel le rapport des constituants est tel que défini dans l'une quelconque des revendications 3 à 5.

11. Composition pharmaceutique comprenant un véhicule pharmaceutiquement acceptable et une quantité efficace d'une formulation granulaire selon l'une quelconque des revendications 1 à 7.

12. Composition selon la revendication 11, dans laquelle le véhicule est un aliment pour animaux.